# EUROPEAN PATENT APPLICATION

(11) **EP 4 566 650 A1**
(43) Date of publication of application: **11.06.2025**
(21) Application number: 23849763.0
(22) Date of filing: 12.06.2023
(51) Int. Cl.: A61M 25/00, A61M 25/10

(54) **BALLOON CATHETER**

(30) Priority: 02.08.2022 JP 2022123322
(71) Applicant: Goodman Co., Ltd., Aichi 460-0008 (JP)
(72) Inventor: OKAMOTO, Mitsumasa, Seto-shi, Aichi 489-0976 (JP); KONDO, Shoma, Seto-shi, Aichi 489-0976 (JP); YAMAMOTO, Shuhei, Seto-shi, Aichi 489-0976 (JP); NAKAMURA, Yuta, Seto-shi, Aichi 489-0976 (JP)
(74) Representative: Epping - Hermann - Fischer
(86) International application number: PCT/JP2023/021750
(87) International publication number: WO 2024/029203

(57) **Abstract**

A shaft body (20) includes a bending region (2R) including at least one bending portion (26) at a section between the first tip end portion (23D) and the first connection portion (226). A stiffness of a first portion (Q1) is a first stiffness (G1). The first portion (Q1) is further to the side of the first tip end portion (23D) than the bending region (2R). A stiffness of a second portion (Q2) is a second stiffness (G2). The second portion (Q2) is overlapping the bending region (2R). A stiffness of a third portion (Q3) is a third stiffness (G3). The third portion (Q3) is further to the side of the first base end portion than the bending region (2R). The second stiffness (G2) is larger than the first stiffness (G1), and the third stiffness (G3) is larger than the second stiffness (G2).

## Description

### Technical Field

The present invention relates to a balloon catheter.

### Background art

A balloon catheter is known as a medical device that dilates a stenotic lesion inside a blood vessel by inflating a balloon. Further, various techniques are proposed for causing the balloon to rapidly reach the vicinity of the stenotic lesion (refer to Patent Literature 1, for example).

### Citation List

### Patent Literature

Patent Literature 1: JP H9-56821 A.

### Summary of Invention

### Problems that the invention is to solve

In the known balloon catheter, it is difficult to cause the balloon to reach the lesion while passing through curved sections of the blood vessel and passing over obstructions inside the blood vessel.

An object of the present invention is to provide a balloon catheter capable of easily passing through curved sections of a blood vessel and passing over obstructions inside the blood vessel.

A balloon catheter according to an aspect of the present invention includes a shaft body and a balloon body. The shaft body extends between a first tip end portion and a first base end portion. The balloon body is connected to the shaft body in the vicinity of the first tip end portion of the shaft body and includes a second tip end portion and a second base end portion. The second tip end portion is connected to a first connection portion being a location of the shaft body separated to the side of the first base end portion with respect to the first tip end portion. The second base end portion is connected to a second connection portion being a location of the shaft body separated to the side of the first base end portion with respect to the first connection portion. The balloon body extends between the second tip end portion and the second base end portion. The shaft body includes a bending region including at least one bending portion at a section between the first tip end portion and the first connection portion. A stiffness of a first portion of the balloon catheter is a first stiffness. The first portion is a portion further to the side of the first tip end portion than the bending region, in an extending direction. The extending direction being a direction along a center of the shaft body. A stiffness of a second portion of the balloon catheter is a second stiffness. The second portion is a portion overlapping the bending region in the extending direction. A stiffness of a third portion of the balloon catheter is a third stiffness. The third portion is a portion further to the side of the first base end portion than the bending region in the extending direction. The second stiffness is larger than the first stiffness, and the third stiffness is larger than the second stiffness.

In the present invention, the stiffness of the second portion overlapping the bending region of the balloon catheter is larger than the stiffness of the first portion, which is the portion further to the side of the first tip end portion than the bending region of the balloon catheter. Note that the larger the stiffness, the more stably the bent state is maintained. Thus, the balloon catheter can stably maintain the state which the shaft body including the second portion is bent at a bending portion of the bending region.

Further, the stiffness of the third portion that is the portion further to the side of the first base end portion than the second portion of the balloon catheter is larger than the stiffness of the first portion and of the second portion. Thus, the balloon catheter realizes favorable pushability. As a result, a user can efficiently push in the balloon catheter that includes the bending portion toward a stenotic lesion. In this way, the user can easily operate the balloon catheter such that the balloon body passes through curved sections of a blood vessel and passes over obstructions in the blood vessel.

According to an aspect of the present invention, the shaft body includes a shaft main body portion having a lumen and a tip provided at an end portion, of the shaft main body portion, closest to the first tip end portion, the tip having a smaller stiffness than that of the shaft main body portion. The bending portion is provided at the shaft main body portion. In this case, the balloon catheter can stably maintain the state in which the shaft body is bent at the bending portion, by forming, at the shaft main body portion, the bending portion having the relatively large stiffness. Further, using the tip, it is possible to suppress damage to an inner wall of the blood vessel by the first tip end portion during movement of the shaft body.

According to an aspect of the present invention, a cover portion covering at least the bending region of the shaft body. In this case, the cover portion can stably maintain the state in which the shaft body is bent at the bending portion of the bending region.

According to an aspect of the present invention, the balloon body includes a balloon configured to inflate and deflate. An element provided at the balloon. A stiffness of the balloon body is larger than a stiffness of the balloon. In this case, the balloon catheter can easily realize, using the element of the balloon body, a relationship in which the stiffness of the third portion (the third stiffness) is larger than the stiffnesses of the first portion and of the second portion (the first stiffness and the second stiffness). Thus, the balloon catheter can realize the favorable pushability using the element of the balloon body.

According to an aspect of the present invention, the element extends between the second tip end portion and the second base end portion. In this case, the balloon catheter can increase the stiffness over a whole region in the extending direction of the balloon body. Thus, the balloon catheter can realize the favorable pushability.

According to an aspect of the present invention, the bending region includes two or more of the bending portions. In this case, the balloon catheter can cause the balloon body to reach the stenotic lesion by adjusting bending directions at the two or more bending portions of the shaft body in accordance with the shape of the curved section of the blood vessel or of the obstruction in the blood vessel.

According to an aspect of the present invention, the first tip end portion of the shaft body is rounded. In this case, when the first tip end portion comes into contact with the obstruction when pushing the balloon catheter into the blood vessel, as a result of the shaft body receiving a force from the obstruction, the first tip end portion of the shaft body changes to an orientation such that the first tip end portion can easily pass over the obstruction. Thus, a user can change the orientation of the first tip end portion of the shaft body without needing to manually rotate the shaft body.

According to an aspect of the present invention, a thickness of an outer side section of the bending portion of the shaft body is thinner than a thickness of an inner side section of the bending portion of the shaft body. In this case, the balloon catheter can stably maintain the state in which the shaft body is bent at the bending portion.

According to an aspect of the present invention, a length of the shaft body in the extending direction from the first tip end portion to the bending portion is equal to or greater than 1 cm. In this case, the user can easily operate the balloon catheter such that the balloon body passes through the curved section of the blood vessel and passes over the obstruction inside the blood vessel, and reaches the stenotic lesion.

### Brief description of the drawings

Fig. 1 is a view showing a balloon catheter 1A.
Fig. 2 is a cross-sectional view in which a tip end portion of the balloon catheter 1A is expanded.
Fig. 3 is a view showing relationships between the stiffness of a first portion Q1, of a second portion Q2, and of a third portion Q3 of the balloon catheter 1A.
Fig. 4 is an explanatory diagram of a first example of operation examples of the balloon catheter 1A.
Fig. 5 is an explanatory diagram of the first example of the operation examples of the balloon catheter 1A.
Fig. 6 is an explanatory diagram of the first example of the operation examples of the balloon catheter 1A.
Fig. 7 is an explanatory diagram of the first example and a second example of the operation examples of the balloon catheter 1A.
Fig. 8 is an explanatory diagram of the first example and the second example of the operation examples of the balloon catheter 1A.
Fig. 9 is an explanatory diagram of the first example and the second example of the operation examples of the balloon catheter 1A.
Fig. 10 is an explanatory diagram of the second example of the operation examples of the balloon catheter 1A.
Fig. 11 is an explanatory diagram of the second example of the operation examples of the balloon catheter 1A.
Fig. 12 is a view showing a balloon catheter 1B.
Fig. 13 is a view showing a balloon catheter 1C.

### Description of Embodiments

Embodiments (balloon catheters 1A to 1C) of a balloon catheter 1 according to the present invention will be explained with reference to the drawings. The referenced drawings are used to describe technical features that can be employed in the present invention. The described configurations and the like of the device are not intended to be limited thereto, and are merely examples for explanation purposes.

### First embodiment (Balloon catheter 1A)

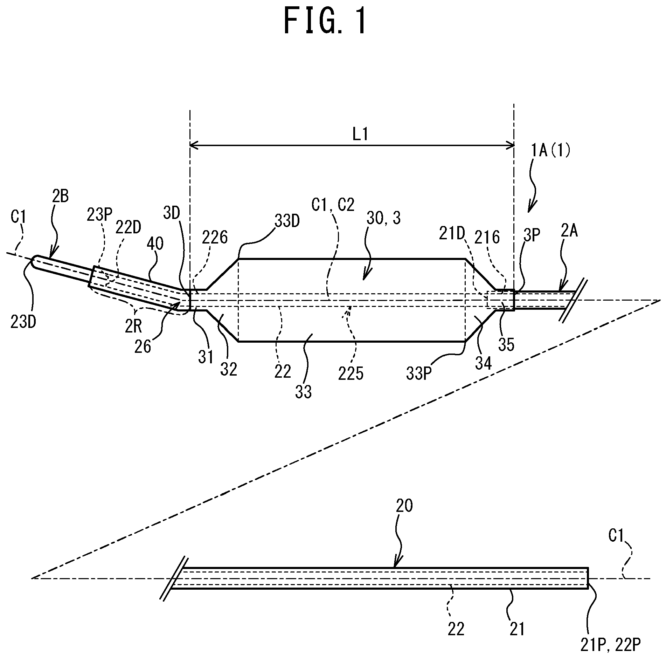

The balloon catheter 1A will be described with reference to Fig. 1 to Fig. 3. The balloon catheter 1A includes a shaft body 20, a balloon body 30, and a cover portion 40. The shaft body 20 is tube-shaped, and includes a bending portion 26 to be described later. The balloon body 30 can be inflated and deflated, and is connected to the shaft body 20. The cover portion 40 covers a bending region 2R, to be described later, of the shaft body 20.

Hereinafter, one side from among both ends of the shaft body 20 will be referred to as a "tip end side". The other side from among both ends of the shaft body 20 will be referred to as a "base end side". A direction extending along the shaft body 20 will be referred to as to as an "extending direction". An axis passing through the center of the shaft body 20 and extending in the extending direction will be referred to as a center axis C1. A radial direction centered on the center axis C1 will simply be referred to as the "radial direction". A cross section cut on a plane orthogonal to the center axis C1 will simply be referred to as a "cross section". In the cross section, the side closer to the center axis C1 in the radial direction will be referred to as an "inner side," and the side further away from the center axis C1 will be referred to as an "outer side." A circumferential direction centered on the center axis C1 will simply be referred to as the "circumferential direction".

### Shaft body 20

The shaft body 20 includes a shaft main body portion 2A and a tip 2B.

As shown in Fig. 1, the shaft main body portion 2A is formed of an outer tube 21 and an inner tube 22, each including a lumen. The outer tube 21 and the inner tube 22 are both flexible. The inner diameter of the outer tube 21 is larger than the outer diameter of the inner tube 22. Apart from a predetermined section on the tip end side, the inner tube 22 is disposed inside the lumen of the outer tube 21. The predetermined section on the tip end side of the inner tube 22 protrudes toward the tip end side from the end portion (hereinafter referred to as a "tip end portion 21D") on the tip end side of the outer tube 21. The end portion (hereinafter referred to as a "tip end portion 22D") on the tip end side of the inner tube 22 is disposed further to the tip end side than the tip end portion 21D of the outer tube 21.

Hereinafter, the predetermined section on the tip end side of the inner tube 22 will be referred to as a "protruding section 225." The end portion on the base end side of the outer tube 21 will be referred to as a "first base end portion 21P". The end portion on the base end side of the inner tube 22 will be referred to as a "first base end portion 22P". A hub is connected to at least the first base end portion 21P. The material of the outer tube 21 and the inner tube 22 is not particularly limited, and a polyamide resin may be used, for example.

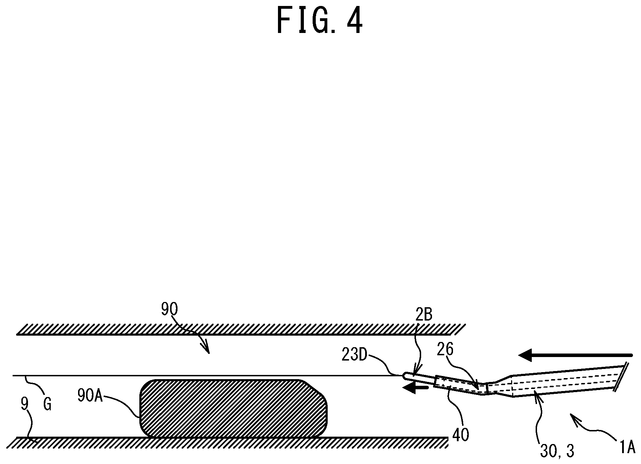

A compressed fluid supplied from the hub passes through a space of the lumen of the outer tube 21 other than the lumen of the inner tube 22. A guide wire G (refer to Fig. 4 and the like) is inserted through the lumen of the inner tube 22.

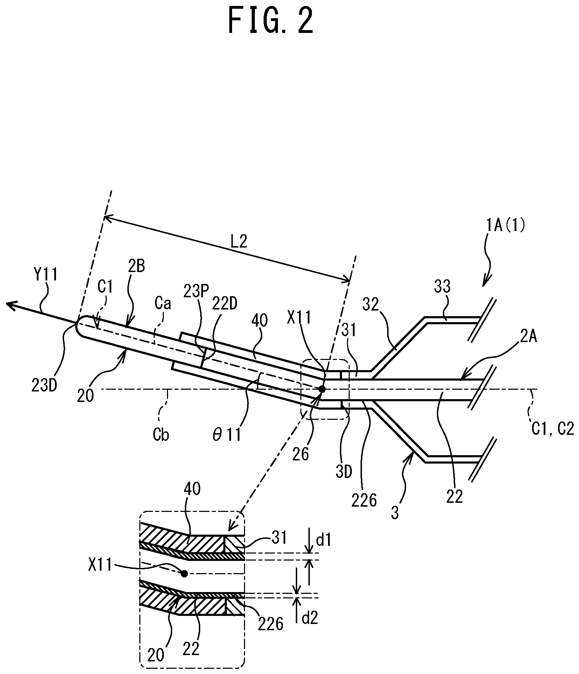

As shown in Fig. 2, the tip 2B is provided at the inner tube 22 of the shaft main body portion 2A. The tip 2B has a rod shape, and a cross-sectional shape of the tip 2B is circular. The outer diameter of the tip 2B is the same as the outer diameter of the inner tube 22. The end portion on the base end side of the tip 2B (hereinafter referred to as a "base end portion 23P") is connected to the tip end portion 22D of the inner tube 22. The tip 2B extends along the extending direction toward the tip end side from the tip end portion 22D. The end portion on the tip end side of the tip 2B will be referred to as a "first tip end portion 23D".

The first tip end portion 23D of the tip 2B has a hemispherical shape, and is rounded. A through hole extending along the extending direction is provided at the center of the tip 2B. The guide wire G is inserted through the through hole. The material of the tip 2B is not particularly limited, and rubber may be used, for example. The stiffness of the tip 2B is smaller than the stiffness of the shaft main body portion 2A. Thus, the tip 2B is softer than the shaft main body portion 2A.

### Balloon body 30

As shown in Fig. 1, the balloon body 30 includes at least a balloon 3. The balloon 3 can change shape between a deflated state and an inflated state, as a result of a change in internal pressure according to whether the compressed fluid is supplied from the hub that is not shown in the drawings. Fig. 1 shows the balloon 3 in the inflated state. On the other hand, a plurality of pleats are formed at the balloon 3 in the deflated state.

The balloon 3 is connected to the shaft body 20 in the vicinity of the first tip end portion 23D of the shaft body 20. A tip end connection portion 31, a tip end coupling portion 32, an inflatable portion 33, a base end coupling portion 34, and a base end connection portion 35 are defined in the balloon 3.

The inflatable portion 33 is tube-shaped and the diameter thereof is substantially the same over the extending direction in the inflated state. The end portion on the tip end side of the inflatable portion 33 will be referred to as a "tip end portion 33D", and the end portion on the base end side will be referred to as a "base end portion 33P". The length of the inflatable portion 33 in the extending direction is 13 mm, for example. The tip end coupling portion 32 extends with a shrinking diameter toward the tip end side from the tip end portion 33D in the inflated state. The base end coupling portion 34 extends with a shrinking diameter toward the base end side from the base end portion 33P in the inflated state. The length of each of the tip end coupling portion 32 and the base end coupling portion 34 in the extending direction is 3 mm, for example.

The tip end connection portion 31 is provided on the opposite side, of the tip end coupling portion 32, from the side at which the tip end coupling portion 32 is coupled to the inflatable portion 33. The tip end connection portion 31 is connected by thermal welding, at a location, of the shaft body 20, separated to the base end side with respect to the first tip end portion 23D, more specifically, at a location separated to the base end side with respect to the tip end portion 22D of the outer surface of the inner tube 22 of the shaft body 20. Hereinafter, the location of the inner tube 22 to which the tip end connection portion 31 of the balloon 3 is connected will be referred to as a "first connection portion 226". The end portion of the tip end connection portion 31 on the opposite side from the side at which the tip end connection portion 31 is connected to the tip end coupling portion 32 will be referred to as a "second tip end portion 3D". The second tip end portion 3D corresponds to the end portion on the tip end side of the balloon 3. The length of the tip end connection portion 31 in the extending direction is 1.5 mm, for example.

The base end connection portion 35 is provided on the opposite side, of the base end coupling portion 34, from the side at which the base end coupling portion 34 is coupled to the inflatable portion 33. The base end connection portion 35 is connected by thermal welding, at a location, of the shaft body 20, separated to the base end side with respect to the first connection portion 226, more specifically, at a location in the vicinity of the tip end portion 21D of the outer surface of the outer tube 21 of the shaft body 20. Hereinafter, the location of the outer tube 21 to which the base end connection portion 35 of the balloon 3 is connected will be referred to as a "second connection portion 216". The end portion of the base end connection portion 35 on the opposite side from the side at which the base end connection portion 35 is connected to the base end coupling portion 34 will be referred to as a "second base end portion 3P". The second base end portion 3P corresponds to the end portion on the base end side of the balloon 3. The length of the base end connection portion 35 in the extending direction is 2.5 mm, for example.

The balloon 3 extends between the second tip end portion 3D and the second base end portion 3P, and covers the protruding section 225 of the inner tube 22 from the outside. The length between the second tip end portion 3D and the second base end portion 3P of the balloon 3 is denoted by L1. The length L1 matches a sum of the respective lengths, in the extending direction, of the tip end connection portion 31, the tip end coupling portion 32, the inflatable portion 33, the base end coupling portion 34, and the base end connection portion 35, and is 23 mm, for example. The material of the balloon 3 is not particularly limited, and a polyamide resin or a polyamide elastomer resin can be used, for example.

An axis extending through the center of the balloon 3 will be referred to as a "center axis C2". The center axis C2 overlaps a part of the center axis C1 extending through the center of the shaft body 20, more specifically, overlaps the center axis C1 at the protruding section 225 of the inner tube 22. As shown in Fig. 2, an axis extending in a straight line to the tip end side along the section at which the center axes C1 and C2 overlap each other will be referred to as a "reference axis Cb".

### Bending portion 26

As shown in Fig. 2, the shaft main body portion 2A of the shaft body 20 is bent at a section slightly further to the tip end side than the first connection portion 226 to which the tip end connection portion 31 of the balloon 3 is connected. Hereinafter, this section will be referred to as the "bending portion 26". The bending portion 26 is positioned between the first tip end portion 23D of the shaft body 20 and the first connection portion 226, more specifically, between the tip end portion 22D of the shaft main body portion 2A of the shaft body 20 and the first connection portion 226. The length of the first tip end portion 23D of the shaft body 20 and the bending portion 26 is denoted by L2. The length L2 is 1 cm, for example. Note that the length L2 is not limited to being 1 cm, and it is sufficient that the length L2 be equal to or longer than 1 cm.

Of the center axis C1 of the shaft body 20, a section further to the tip end side than the bending portion 26 will be referred to as a "section axis Ca". The section axis Ca is inclined with respect to the reference axis Cb. A position of an intersection point X11 between the section axis Ca and the reference axis Cb is aligned with the bending portion 26 in the extending direction. An angle formed between the section axis Ca and the reference axis Cb will be referred to as a "bending angle θ11". The bending angle θ11 is 30°, for example. A direction extending along the extending direction through the first tip end portion 23D of the tip 2B and toward the opposite side from the side of the bending portion 26 with respect to the first tip end portion 23D will be referred to as a "bending direction Y11".

In the bending portion 26 of the shaft main body portion 2A, a thickness differs between an inner side section and an outer side section. The inner side section indicates a section of the bending portion 26 closer to a center of the bend. The outer side section indicates a section of the bending portion 26 further from the center of the bend. The thickness of the inner side section is denoted by d1, and the thickness of the outer side section is denoted by d2. In this case, the thickness d2 is thinner than the thickness d1 (d1 > d2). Thus, in the shaft main body portion 2A, the inner side section is thicker than the outer side section.

### Cover portion 40

As shown in Fig. 1 and Fig. 2, of the shaft body 20, the cover portion 40 is provided further to the tip end side than the first connection portion 226 to which the tip end connection portion 31 of the balloon 3 is connected. The cover portion 40 is tube-shaped. The cover portion 40 covers, from outside, a section of the shaft body 20 including at least the bending portion 26. The end portion on the base end side of the cover portion 40 is in contact with the second tip end portion 3D of the balloon 3. The end portion on the tip end side of the cover portion 40 is positioned further to the tip end side than the tip end portion 22D of the shaft main body portion 2A and the base end portion 23P of the tip 2B. Thus, the cover portion 40 covers a part of the tip end side of the shaft main body portion 2A of the shaft body 20, and a part of the base end side of the tip 2B, respectively. Hereinafter, a region of the shaft body 20 covered by the cover portion 40 will be referred to as a bending region 2R. The bending portion 26 is included in the bending region 2R.

The material of the cover portion 40 is not particularly limited, and a polyether block amide can be used, for example. The thickness of the cover portion 40 is larger than the thickness of the inner tube 22. Thus, the stiffness of the cover portion 40 is larger than the stiffness of the shaft body 20.

Note that the thickness of the cover portion 40 need not necessarily be uniform. For example, the thickness of the cover portion 40 may become gradually larger from the end portion on the tip end side to the end portion on the base end side. In this way, the stiffness of the cover portion 40 may become gradually larger from the end portion on the tip end side to the end portion on the base end side.

### Stiffness of balloon catheter 1A

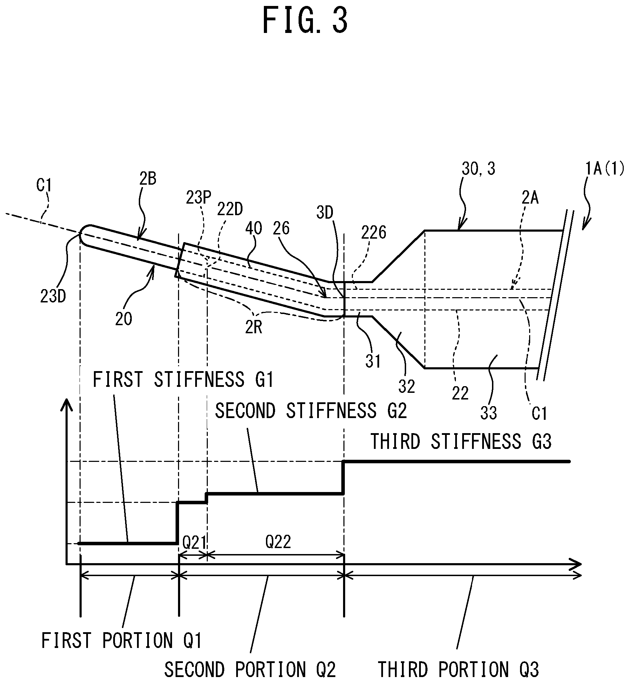

As shown in Fig. 3, three portions (the first portion Q1, the second portion Q2, and the third portion Q3) are defined in the balloon catheter 1A. The first portion Q1 is a portion of the balloon catheter 1A further to the tip end side than the bending region 2R in the extending direction. A part of the tip 2B of the shaft body 20 is included in the first portion Q1. The second portion Q2 is a portion overlapping the bending region 2R of the balloon catheter 1A in the extending direction. A part of the shaft main body portion 2A of the shaft body 20, a part of the tip 2B, and the cover portion 40 are included in the second portion Q2. The third portion Q3 is a portion of the balloon catheter 1A further to the base end side than the bending region 2R in the extending direction. A part of the shaft main body portion 2A, and the balloon 3 are included in the third portion Q3.

The stiffness of the first portion Q1 of the balloon catheter 1A will be referred to as a "first stiffness G1". The stiffness of the second portion Q2 of the balloon catheter 1A will be referred to as a "second stiffness G2". The stiffness of the third portion Q3 of the balloon catheter 1A will be referred to as a "third stiffness G3". In this case, the second stiffness G2 is larger than the first stiffness G1, and the third stiffness G3 is larger than the second stiffness G2 (G1 < G2 < G3).

Note that each of the stiffnesses of the second portion Q2 and the third portion Q3 (the second stiffness G2 and the third stiffness G3) is not uniform in the extending direction. For example, in the extending direction, the second portion Q2 having the second stiffness G2 includes a portion Q21 including a part of the tip 2B and the cover portion 40, and a portion Q22 including a part of the shaft main body portion 2A and the cover portion 40. Thus, the stiffnesses of the portion Q21 and the portion Q22 are different and the second stiffness G2 is not uniform. Further, although not shown in the drawings, in the extending direction, the third portion Q3 includes a portion including the balloon 3 and the inner tube 22, a portion including the balloon 3, the inner tube 22, and the outer tube 21, and a portion including the inner tube 22 and the outer tube 21. Thus, the stiffnesses of each of the portions are different and the third stiffness G3 is not uniform. On the other hand, only the tip 2B is included in the first portion Q1, and the first stiffness G1 and the stiffness of the tip 2B match each other. Thus, the first stiffness G1 is uniform in the extending direction.

### Manufacturing method of balloon catheter 1A

The balloon catheter 1A is manufactured using the following method. A bent rod-shaped mandrel is prepared, and is inserted into the lumen of the inner tube 22. In this state, the inner tube 22 is bent. Next, the outer tube 21 is disposed around the outside of the inner tube 22, and the balloon 3 is disposed at the protruding section 225 of the inner tube 22.

The outer tube 21, the inner tube 22, and the balloon 3 are mounted to a welder device and are heated. In this way, the tip end connection portion 31 of the balloon 3 is connected to the first connection portion 226 of the inner tube 22. Further, the base end connection portion 35 of the balloon 3 is connected to the second connection portion 216 of the outer tube 21.

Next, the tip end portion 22D of the inner tube 22 is connected to the tip 2B, and the shaft body 20 is formed. Next, the portion of the shaft body 20 including the bending portion 26 is covered by the cover portion 40, and the cover portion 40 is thermally welded to the shaft body 20 by heating. The balloon catheter 1A is manufactured in the above-described manner.

### Operation examples

A first example of operation examples of the balloon catheter 1A will be described with reference to Fig. 4 to Fig. 9. A case will be exemplified in which the balloon catheter 1A is used for dilating a stenotic lesion (not shown in the drawings) that has occurred in part of an inner wall of a vascular channel 9. Further, in the vascular channel 9, an obstruction 90A that is a calcified inner wall or is a stent or the like located in the vascular channel 9, is located at a proximal side of the stenotic lesion. The diameter of the lumen of the vascular channel 9 becomes smaller at the location at which the obstruction 90A is located. Hereinafter, the portion of the lumen of the vascular channel 9 at which the diameter is smaller due to the obstruction 90A will be referred to as a "narrowed lumen 90".

First, the guide wire G is inserted into the vascular channel 9. The balloon catheter 1A including the balloon 3 in the deflated state is prepared. Of the balloon catheter 1A, at least a portion including the balloon 3 is disposed inside the vascular channel 9. The guide wire G is inserted into the shaft body 20 of the balloon catheter 1A.

As shown in Fig. 4, as a result of the shaft body 20 (refer to Fig. 1) of the balloon catheter 1A being operated, the balloon catheter 1A is pushed into the vascular channel 9 along the guide wire G. In a state in which the balloon 3 is disposed at a front end in a movement direction, the balloon catheter 1A moves to the distal side in the vascular channel 9, toward the stenotic lesion.

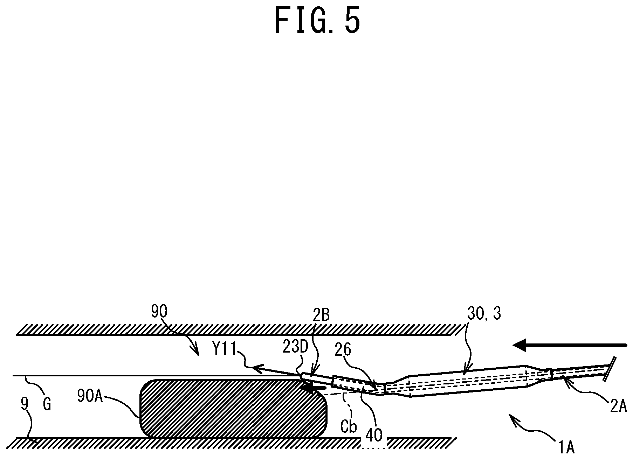

As shown in Fig. 5, the tip 2B of the balloon catheter 1A comes into contact with the obstruction 90A before the balloon 3 reaches the stenotic lesion. Note that, in the first example, the tip 2B comes into contact with the obstruction 90A while the reference axis Cb of the balloon catheter 1A is oriented in a direction intersecting the obstruction 90A. The movement of the balloon catheter 1A to the distal side is temporarily restricted by the obstruction 90A.

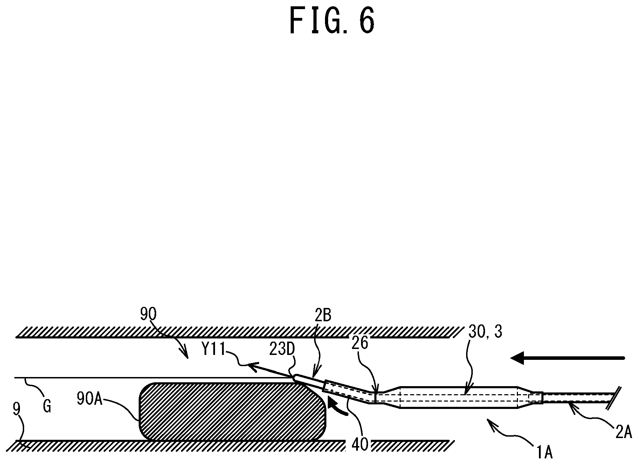

The shaft body 20 is operated, and the balloon catheter 1A is pushed further into the vascular channel 9. Here, since the first tip end portion 23D of the tip 2B of the balloon catheter 1A is rounded, as shown in Fig. 6, the tip 2B does not become caught up on the obstruction 90A. Further, since the shaft main body portion 2A is bent at the bending portion 26, in accordance with the force pushing the balloon catheter 1A into the vascular channel 9, the balloon catheter 1A rotates such that the bending direction Y11 is oriented in a direction separating from the obstruction 90A.

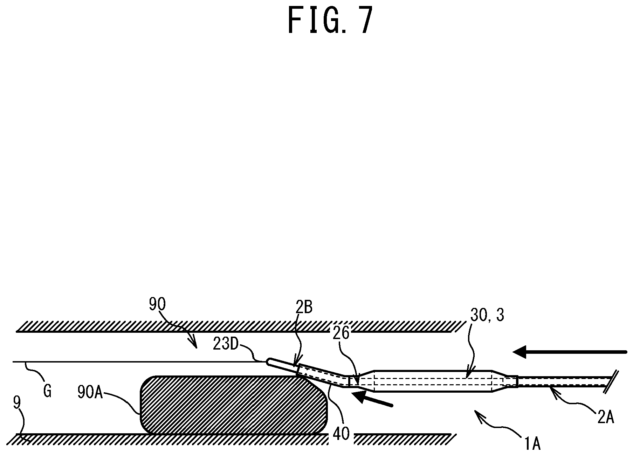

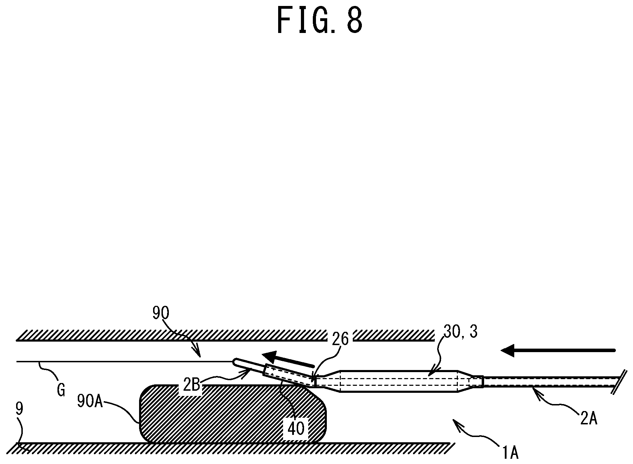

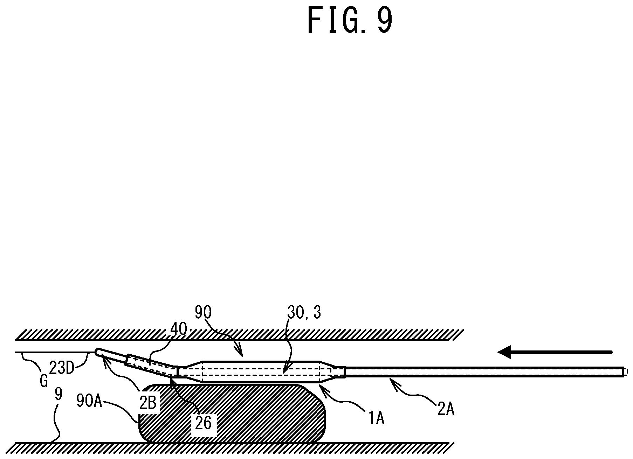

Thereafter, in accordance with the balloon catheter 1A being pushed further into the vascular channel 9, as shown in Fig. 7, the tip 2B and the balloon 3 move toward the narrowed lumen 90. As shown in Fig. 8, the tip 2B passes through the narrowed lumen 90 and moves to the distal side. As shown in Fig. 9, in accordance with the balloon catheter 1A being pushed further into the vascular channel 9, the balloon 3 passes over the obstruction 90A, passes through the narrowed lumen 90, and moves to the distal side.

After the balloon 3 has reached the stenotic lesion inside the vascular channel 9, the movement of the balloon catheter 1A is stopped. Next, the supply of the compressed fluid to the balloon 3 is started, and the balloon 3 enters into the inflated state. The balloon 3 in the inflated state dilates the stenotic lesion.

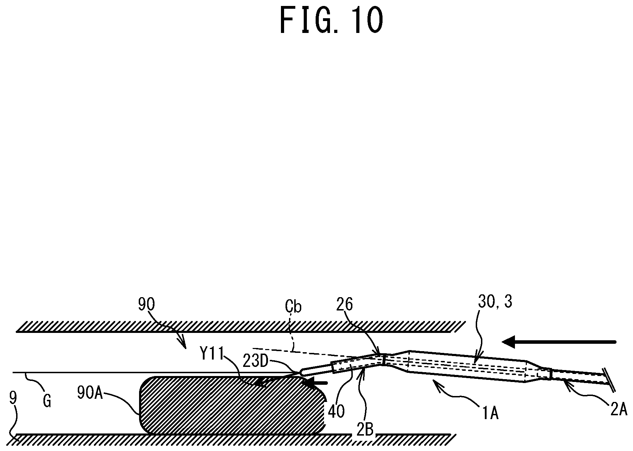

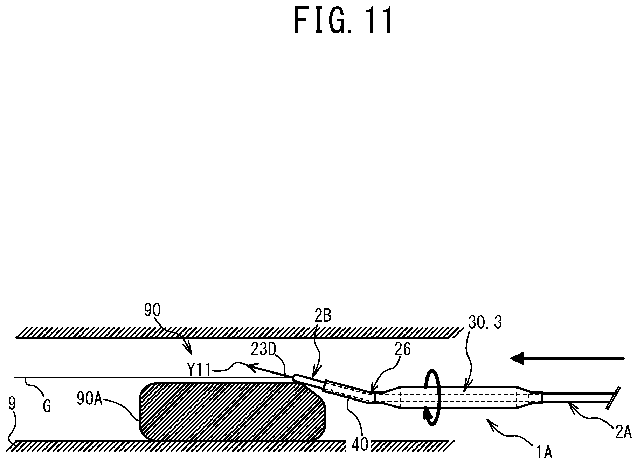

A second example of the operation examples of the balloon catheter 1A will be described with reference to Fig. 10 and Fig. 11. A point of difference from the first example is the arrangement of the reference axis Cb when the tip 2B has come into contact with the obstruction 90A. As shown in Fig. 10, in a state in which the tip 2B is in contact with the obstruction 90A, the reference axis Cb of the balloon catheter 1A does not intersect the obstruction 90A.

The shaft body 20 is operated, and the balloon catheter 1A is pushed further into the vascular channel 9. Note that, since the first tip end portion 23D of the tip 2B of the vascular channel 9 is rounded, the tip 2B does not become caught up on the obstruction 90A. Further, since the shaft main body portion 2A is bent at the bending portion 26, in accordance with the force pushing the balloon catheter 1A into the vascular channel 9, the balloon catheter 1A rotates around the center axis C1 as shown in Fig. 11. The bending direction Y11 changes to an orientation separating from the obstruction 90A.

The behavior of the balloon catheter 1A thereafter is the same as shown in Fig. 7 to Fig. 9 of the first example. In accordance with the balloon catheter 1A being pushed further in, the tip 2B and the balloon 3 move toward the narrowed lumen 90 (refer to Fig. 7). The tip 2B passes through the narrowed lumen 90 and moves to the distal side (refer to Fig. 8). In accordance with the balloon catheter 1A being pushed further in, the balloon 3 passes over the obstruction 90A, passes through the narrowed lumen 90, and moves to the distal side (refer to Fig. 9). After the balloon 3 has reached the stenotic lesion inside the vascular channel 9, the balloon 3 enters into the inflated state and dilates the stenotic lesion.

### Actions and effects of first embodiment

The shaft body 20 of the balloon catheter 1A is bent at the bending portion 26. Thus, when the tip 2B has come into contact with the obstruction 90A, a user can easily orient the bending direction Y11 in the direction separating from the obstruction 90A, simply by performing the operation of pushing the balloon catheter 1A into the vascular channel 9. Thus, the balloon catheter 1A can easily cause the balloon 3 to reach the stenotic lesion on the distal side of the obstruction 90A.

The stiffness (the second stiffness G2) of the second portion Q2 overlapping the bending region 2R of the shaft body 20 is larger than the stiffness (the first stiffness G1) of the first portion Q1 that is the portion of the shaft body 20 further to the side of the first tip end portion 23D than the bending region 2R. Note that the larger the stiffness, the more stably the shaft body 20 is maintained in the bent state. In other words, the shaft body 20 is stably maintained in the state in which the bending portion 26 of the second portion Q2 is bent, and does not easily return to a state of extending in a straight line. Thus, the balloon catheter 1A can stably maintain, over a long period, a function of the balloon 3 being able to pass over the obstruction 90A.

Further, the stiffness (the third stiffness G3) of the third portion Q3, which is the portion further to the side of the first base end portions 21P and 22P than the bending region 2R of the shaft body 20, is even larger than the second stiffness G2. Thus, the balloon catheter 1A can realize favorable pushability when being pushed into the vascular channel 9. Thus, the user can efficiently push the balloon catheter 1A having the shaft body 20 including the bending portion 26 toward the stenotic lesion inside the vascular channel 9. As a result, the user can easily operate the balloon catheter 1A such that the balloon 3 passes over the obstruction 90A inside the vascular channel 9 and reaches the stenotic lesion.

The shaft body 20 includes the shaft main body portion 2A provided with the bending portion 26, and the tip 2B having the smaller stiffness than the shaft main body portion 2A. In other words, in the balloon catheter 1A, it is possible to provide the bending portion 26 at the shaft main body portion 2A having the relatively large stiffness. Thus, the balloon catheter 1A can stably maintain the state in which the shaft main body portion 2A is bent at the bending portion 26. Further, as a result of making the stiffness of the tip 2B relatively small, the balloon catheter 1A can suppress the first tip end portion 23D of the tip 2B from damaging the inner wall of the vascular channel 9 when moving the shaft body 20.

The balloon catheter 1A includes the cover portion 40 that covers the portion of the shaft body 20 including the bending portion 26. The cover portion 40 can stably maintain the state in which the shaft body 20 is bent at the bending portion 26.

When the first tip end portion 23D has come into contact with the obstruction 90A when the balloon catheter 1A has been pushed into the vascular channel 9, the tip 2B receives a force from the obstruction 90A. Here, the first tip end portion 23D of the tip 2B is rounded. In this case, the balloon catheter 1A automatically changes the orientation of the bending direction Y11 such that the balloon 3 can easily pass over the obstruction 90A. Thus, the user can change the orientation of the bending direction Y11 such that the balloon 3 can easily pass over the obstruction 90A, without needing to operate the shaft body 20.

In the shaft main body portion 2A, the thickness d2 of the outer side section of the bending portion 26 is thinner than the thickness d1 of the inner side section of the bending portion 26. In this case, the balloon catheter 1A can stably maintain the state in which the shaft body 20 is bent at the bending portion 26.

The length L2 in the extending direction from the first tip end portion 23D of the shaft body 20 to the bending portion 26 is equal to or greater than 1 cm. In this case, the user can easily operate the balloon catheter 1A such that the balloon 3 passes over the obstruction 90A inside the vascular channel 9 and reaches the stenotic lesion.

### Special notes relating to first embodiment

Each of the values of the length L1 between the second tip end portion 3D and the second base end portion 3P of the balloon 3, of the length L2 between the first tip end portion 23D of the shaft body 20 and the bending portion 26, and of the bending angle θ11 is one example thereof, and each may be another value. The section between the first tip end portion 23D of the shaft body 20 and the bending portion 26 may be curved. The bending portion 26 need not necessarily be provided in the vicinity of the first connection portion 226 of the inner tube 22, to which the tip end connection portion 31 of the balloon 3 is connected. For example, the bending portion 26 may be provided in the vicinity of the tip end portion 22D of the inner tube 22.

A member may be further provided for increasing the stiffness of the third portion Q3 of the balloon catheter 1A. For example, an auxiliary body extending between the second tip end portion 3D of the balloon 3 and the first base end portions 21P and 22P of the shaft body 20 may be provided at the shaft body 20. The auxiliary body may have a wire shape, or may be an annular braided tube.

The bending portion 26 may be provided at the tip 2B of the shaft body 20. The stiffnesses of each of the shaft main body portion 2A and the tip 2B of the shaft body 20 may be the same as each other. The first tip end portion 23D of the tip 2B need not necessarily be rounded, and may be angular. The shaft body 20 need not necessarily include the tip 2B and may include only the shaft main body portion 2A. In this case, the shaft main body portion 2A may protrude further to the tip end side than the end portion on the tip end side of the cover portion 40. Furthermore, in this case, the end portion on the tip end side of the shaft main body portion 2A may be angular, or may be rounded.

The cover portion 40 may cover only a section of the inner tube 22 of the shaft body 20 further to the tip end side than the first connection portion 226 to which the tip end connection portion 31 of the balloon 3 is connected, and need not necessarily cover a part of the tip 2B. The end portion on the base end side of the cover portion 40 may cover the tip end connection portion 31 of the balloon 3 from the outside. The thickness of the end portion on the tip end side of the cover portion 40 may become thinner the further toward the tip end, such that a step is not formed between the cover portion 40 and the tip 2B. The cover portion 40 may have a radiopaque marker function. In this case, the user can perform the operation to push in the balloon catheter 1A while ascertaining the position of the bending portion 26 by verifying the position of the cover portion 40 inside the body.

The balloon catheter 1A need not necessarily include the cover portion 40. In this case, of the inner tube 22 of the shaft body 20, the section further to the tip end side than the first connection portion 226 to which the tip end connection portion 31 of the balloon 3 is connected may be defined as the bending region 2R including the bending portion 26.

In the shaft main body portion 2A, the thickness d1 of the inner side section of the bending portion 26 may be thinner than the thickness d2 of the outer side section of the bending portion 26, or the thicknesses d1 and d2 may be the same as each other.

In the first example and the second example described as the operation examples, the process of the balloon 3 passing over the obstruction 90A inside the vascular channel 9 is described. In contrast to this, for example, even when the balloon catheter 1A is inserted into the curved vascular channel 9, by the same operation as in the first example and the second example, the balloon 3 can pass over the curved vascular channel 9 and can reach the stenotic lesion.

### Second embodiment (balloon catheter 1B)

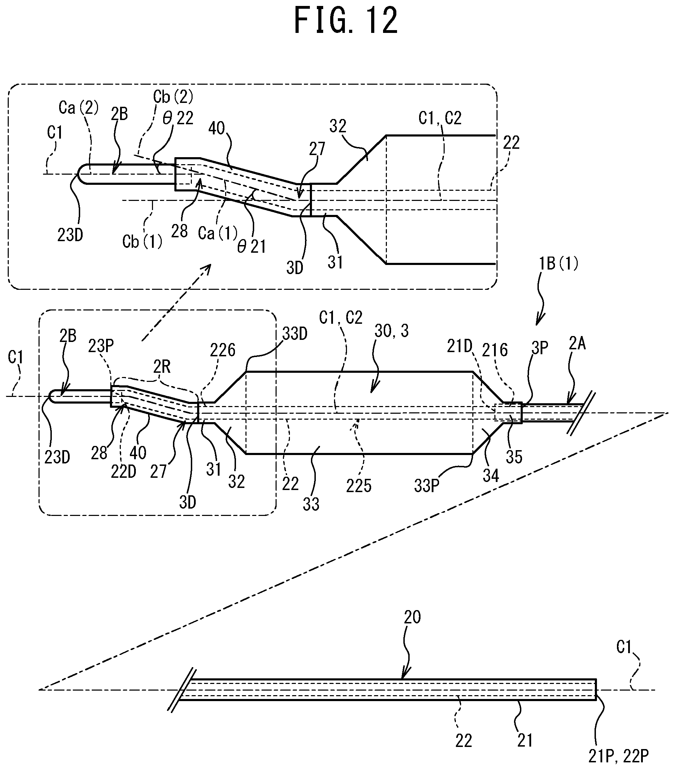

A balloon catheter 1B will be described with reference to Fig. 12. The balloon catheter 1B differs from the balloon catheter 1A in that the shaft main body portion 2A of the shaft body 20 includes bending portions 27 and 28.

The shaft main body portion 2A of the shaft body 20 is bent at a section (hereinafter referred to as the "bending portion 27") that is slightly further to the tip end side of the first connection portion 226 to which the tip end connection portion 31 of the balloon 3 is connected and at a section (hereinafter referred to as the bending portion 28") that is slightly further to the base end side than the tip end portion 22D to which the tip 2B is connected. Both of the bending portions 27 and 28 are positioned between the first tip end portion 23D of the shaft body 20 and the first connection portion 226, more specifically, between the tip end portion 22D of the shaft main body portion 2A of the shaft body 20 and the first connection portion 226.

Of the center axis C1 of the shaft body 20, a section sandwiched between the bending portions 27 and 28 will be referred to as a "section axis Ca (1)". Of the center axis C1 of the shaft body 20, a section further to the tip end side than the bending portion 28 will be referred to as a "section axis Ca (2)". An axis extending in a straight line to the tip end side along the section at which the center axis C2 extending through the center of the balloon 3 overlaps the center axis C1 will be referred to as a "reference axis Cb (1)". An axis extending in a straight line to the tip end side along the section axis Ca (1) will be referred to as a "reference axis Cb (2)". The section axis Ca (1) is inclined with respect to the reference axis Cb (1). The angle between the reference axis Cb (1) and the section axis Ca (1) will be referred to as a "bending angle θ21". The bending angle θ21 is 30°, for example. The section axis Ca (2) is inclined with respect to the reference axis Cb (2). An angle between the reference axis Cb (2) and the section axis Ca (2) will be referred to as a "bending angle θ22". The bending angle θ22 is 30°, for example. The bending angles θ21 and θ22 are substantially the same as each other.

### Actions and effects of second embodiment

The shaft body 20 of the balloon catheter 1B includes the two bending portions (the bending portions 27 and 28). In this case, even when the obstruction 90A of the vascular channel 9 has a complex shape, the balloon catheter 1B can be easily operated to pass over the obstruction 90A such that the balloon 3 reaches the stenotic lesion. Further, even when a plurality of the curved sections of the vascular channel 9 are continuous, the user can operate the balloon catheter 1B to pass through the plurality of curved sections such that the balloon 3 reaches the stenotic lesion.

### Special notes relating to second embodiment

Each of the values of the bending angles θ21 and θ22 is one example thereof, and each may be another value. The bending angles θ21 and θ22 may be different from each other. The number of the bending portions provided in the shaft body 20 is not limited to two and may be three or more.

### Third embodiment (balloon catheter 1C)

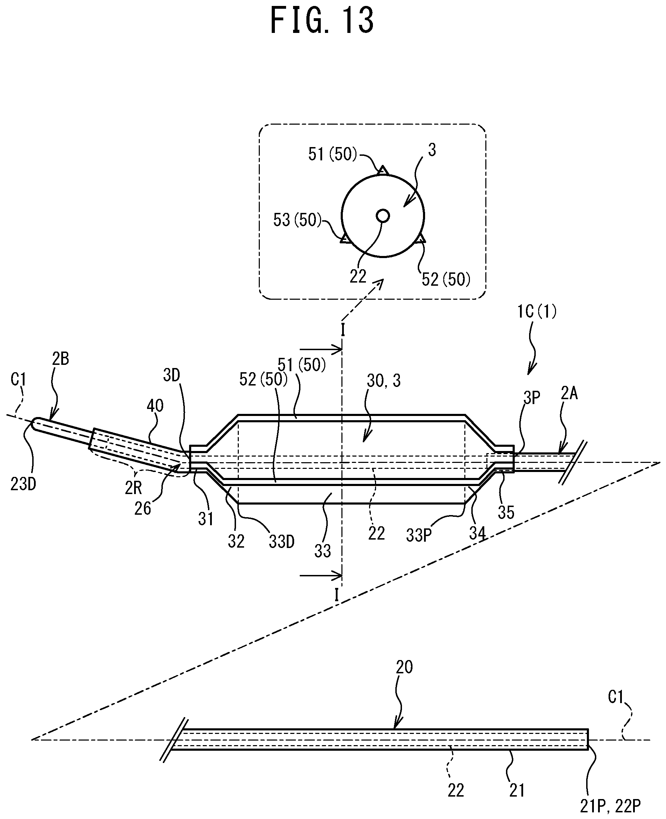

A balloon catheter 1C will be described with reference to Fig. 13. The balloon catheter 1C differs from the balloon catheter 1A in that the balloon catheter 1C includes the balloon body 30 that has elements 51, 52, and 53 (hereinafter referred to as an "element 50" or "elements 50" when no respective distinction is made therebetween) in addition to the balloon 3.

The element 50 has a triangular column shape and extends in the extending direction along the outer surface of the balloon 3. The elements 51, 52, and 53 are disposed at equal intervals in the circumferential direction along the outer surface of the balloon 3. One of three side surfaces of each of the element 50 is in contact with the outer surface of the balloon 3. The width of the element 50 in the circumferential direction becomes narrower toward the outer side, and the outer side thereof is pointed. The stiffness of the element 50 is larger than the stiffness of the balloon 3.

The position of the end portion on the tip end side of the element 50 matches the position of the second tip end portion 3D of the balloon 3 in the extending direction. The position of the end portion on the base end side of the element 50 matches the position of the second base end portion 3P of the balloon 3 in the extending direction. The element 50 extends between the second tip end portion 3D and the second base end portion 3P of the balloon 3.

When the balloon 3 that has reached the stenotic lesion of the vascular channel 9 is inflated, the balloon catheter 1C causes the elements 50 to bite into the stenotic lesion. In this way, the balloon catheter 1C can suppress the position of the balloon 3 from becoming displaced with respect to the stenotic lesion when the balloon 3 is inflated. The balloon catheter 1C can cut the stenotic lesion using the elements 50. In this way, the balloon catheter 1C can efficiently dilate the stenotic lesion in accordance with the inflation of the balloon 3.

### Actions and effects of third embodiment

The balloon catheter 1C can increase the stiffness of a section overlapping the elements 50, of the third portion Q3 (refer to Fig. 3) that is the portion further to the base end side than the bending region 2R. In other words, in the balloon catheter 1C, a configuration can be easily realized, using the elements 50 of the balloon body 30, in which the stiffness of the third portion Q3 is larger than that of the first portion Q1 (refer to Fig. 3) that is the portion further to the tip end side than the bending region 2R and of the second portion Q2 (refer to Fig. 3) that is the portion overlapping the bending region 2R. Thus, using the elements 50 of the balloon body 30, the balloon catheter 1C can realize the favorable pushability.

The elements 50 can increase the stiffness over the region between the second tip end portion 3D and the second base end portion 3P of the balloon 3. Thus, simultaneously with being able to suppress the balloon 3 from deforming when the balloon catheter 1C is pushed into the vascular channel 9, the favorable pushability can be realized using the elements 50.

### Special notes relating to third embodiment

The number of the elements 50 is not limited to 3, and may be any one of 1, 2, or 4 or more. The element 50 may extend between the tip end portion 33D and the base end portion 33P of the inflatable portion 33 of the balloon 3. Alternatively, the element 50 may extend further to the tip end side than the second tip end portion 3D. In this case, the end portion on the tip end side of the element 50 may be disposed in the vicinity of the bending portion 26. The stiffness of the element 50 may be the same as the stiffness of the balloon 3, or may be smaller than the stiffness of the balloon 3.

The element 50 may be provided on the inner surface of the balloon. In this case, the outer surface of the balloon 3 is smooth and need not necessarily protrude to the outer side at a section at which the element 50 is provided.

Of the balloon catheter 1C, a member may be additionally provided for increasing the stiffness of the portion further to the base end side than the balloon 3. Example, an auxiliary body may be provided at a portion, of the shaft body 20, further to the base end side than the second base end portion 3P of the balloon 3. The auxiliary body may have a wire shape, or may be an annular braided tube.

The element 50 may be formed of the same material as the balloon 3. In this case, the stiffness of the element 50 may be increased by causing a state of the molecular orientation of the element 50 to be different from that of the balloon 3.

## Claims

1. A balloon catheter comprising:
a shaft body extending between a first tip end portion and a first base end portion; and
a balloon body connected to the shaft body in the vicinity of the first tip end portion of the shaft body and including a second tip end portion and a second base end portion,
the second tip end portion being connected to a first connection portion being a location of the shaft body separated to the side of the first base end portion with respect to the first tip end portion,
the second base end portion being connected to a second connection portion being a location of the shaft body separated to the side of the first base end portion with respect to the first connection portion, and
the balloon body extending between the second tip end portion and the second base end portion, wherein
the shaft body includes a bending region including at least one bending portion at a section between the first tip end portion and the first connection portion,
a stiffness of a first portion of the balloon catheter is a first stiffness,
the first portion being a portion further to the side of the first tip end portion than the bending region, in an extending direction, and
the extending direction being a direction along a center of the shaft body,
a stiffness of a second portion of the balloon catheter is a second stiffness, the second portion being a portion overlapping the bending region in the extending direction,
a stiffness of a third portion of the balloon catheter is a third stiffness, the third portion being a portion further to the side of the first base end portion than the bending region in the extending direction, and
the second stiffness is larger than the first stiffness, and the third stiffness is larger than the second stiffness.

2. The balloon catheter according to claim 1, wherein
the shaft body includes
a shaft main body portion having a lumen, and
a tip provided at an end portion, of the shaft main body portion, closest to the first tip end portion, the tip having a smaller stiffness than that of the shaft main body portion, and
the bending portion is provided at the shaft main body portion.

3. The balloon catheter according to claim 1, further comprising:
a cover portion covering at least the bending region of the shaft body.

4. The balloon catheter according to claim 1, wherein
the balloon body includes
a balloon configured to inflate and deflate, and
an element provided at the balloon, and
a stiffness of the balloon body is larger than a stiffness of the balloon.

5. The balloon catheter according to claim 4, wherein
the element extends between the second tip end portion and the second base end portion.

6. The balloon catheter according to claim 1, wherein
the bending region includes two or more of the bending portions.

7. The balloon catheter according to claim 1, wherein
the first tip end portion of the shaft body is rounded.

8. The balloon catheter according to claim 1, wherein
a thickness of an outer side section of the bending portion of the shaft body is thinner than a thickness of an inner side section of the bending portion of the shaft body.

9. The balloon catheter according to claim 1, wherein
a length of the shaft body in the extending direction from the first tip end portion to the bending portion is equal to or greater than 1 cm.
